# EUROPÄISCHE PATENTSCHRIFT

(11) **EP 3 825 017 B1**
(45) Veröffentlichungstag und Bekanntmachung des Hinweises auf die Patenterteilung: **26.07.2023**
(21) Anmeldenummer: 19210187.1
(22) Anmeldetag: 19.11.2019
(51) Int. Cl.: B08B 15/02

(54) **VERARBEITUNGSSYSTEM FÜR PULVER UND VERFAHREN ZUR DEKONTAMINATION EINES SOLCHEN VERARBEITUNGSSYSTEMS**
POWDER PROCESSING SYSTEM AND METHOD FOR DECONTAMINATING SUCH A PROCESSING SYSTEM
SYSTÈME DE TRAITEMENT POUR POUDRE ET PROCÉDÉ DE DÉCONTAMINATION D'UN TEL SYSTÈME DE TRAITEMENT

(43) Veröffentlichungstag der Anmeldung: 26.05.2021
(73) Patentinhaber: Harro Höfliger Verpackungsmaschinen GmbH, 71573 Allmersbach im Tal (DE)
(72) Erfinder: Rau, Oliver, 71573 Allmersbach im Tal (DE); Brugger, Bernhard, 71573 Allmersbach im Tal (DE); Jeschke, Michael, 71573 Allmersbach im Tal (DE); Höpfer, Jonas, 71573 Allmersbach im Tal (DE); Wurst, Reiner, 71573 Allmersbach im Tal (DE)
(74) Vertreter: Zurhorst, Stefan

(56) Entgegenhaltungen:
- EP-A1- 2 883 526
- EP-A2- 0 759 464
- CH-A- 300 425
- DE-U1-202006 003 844
- JP-A- 2005 331 862

## Beschreibung

Die Erfindung betrifft ein Verarbeitungssystem für insbesondere pharmazeutische Pulver mit den Merkmalen nach dem Oberbegriff des Anspruchs 1 sowie ein Verfahren zur Dekontamination eines solchen Verarbeitungssystems.

In der pharmazeutischen Industrie werden zum Teil hochwirksame Pulver verarbeitet, indem sie beispielsweise in harte Gelatinekapseln abgefüllt oder zu Tabletten gepresst werden. Solche hochwirksamen Pulver können in entsprechender Konzentration eine toxische Wirkung ausüben. Bei der Verarbeitung solcher Pulver muss deshalb sichergestellt werden, dass der Maschinenbediener und die Umwelt nicht übermäßig belastet oder gar gefährdet werden.

Nach dem Stand der Technik werden solche Pulver in isolierten Räumen verarbeitet. Eine entsprechende Verarbeitungsvorrichtung ist in einem dicht von einem Gehäuse umschlossenen Produktionsraum angeordnet. Derartige Gehäuse werden auch als Containment bezeichnet. Derweil Containment-Verarbeitungssysteme während des gewöhnlichen Betriebes einen zuverlässigen Schutz der Umgebung sicherstellen, besteht eine weitere Hausforderung darin, das geschlossene Gehäuse bei Bedarf auch öffnen zu können. Problematisch ist die Kontamination des inneren Produktionsraumes und der darin angeordneten Maschinenteile mit unvermeidlichen Pulverrückständen.

Beispielsweise bei einer Produktionsunterbrechung oder nach Abschluss eines Produktionszyklus ist vor dem Öffnen des Gehäuses eine intensive Reinigung zur Entfernung der Pulverrückstände erforderlich.

Eine Möglichkeit hierzu besteht nach dem Stand der Technik in einer intensiven Spülung mit Wasser ggf. unter Zusatz von Reinigungsmitteln. Das abfließende Spülwasser wird auf Pulverbestandteile untersucht. Erst wenn eine hinreichend geringe Konzentration festgestellt wird, darf die Gehäusetür für den Zugriff auf den Produktionsraum geöffnet werden. Ein solcher Prozess wird als "Washing in Place" (WiP) bezeichnet.

Das WiP-Verfahren erfordert speziell ausgestaltete Maschinen mit entsprechend geschlossenen Gehäusen. Maschine und Gehäuse müssen absolut dicht insbesondere gegen das zum Einsatz kommende Spülwasser sein, damit kein kontaminiertes Spülwasser austreten kann. Bei der Vielzahl der beweglichen Teile ist dies schwierig zu realisieren, da nicht nur das Gehäuse einschließlich seiner Tür, sondern auch die beweglichen Teile der Verarbeitungsvorrichtung wie Wellenlager, Betätigungsdurchführungen oder dgl. zuverlässig abgedichtet werden müssen. Der Einsatz von Standardmaschinen verbietet sich deshalb. Die Anschaffung und der Betrieb von WiP-fähigen Maschinen hingegen bedeuten hohe Investitions- und Betriebskosten sowie eine unflexible Produktion. Zusätzlich entstehen auch hohe Entsorgungskosten für das Spülwasser.

Als kostengünstige Alternative zum WiP-Verfahren wird unter bestimmten Umständen das Binden des Pulvers durch einen Sprühnebel akzeptiert. Hierbei kommt eine Zweistoffdüse zum Einsatz, in der Wasser mittels Druckluft zerstäubt wird, um damit die kontaminierten Oberflächen zu benetzen bzw. zu befeuchten. An der Oberfläche anhaftende Pulverrückstände werden durch die Feuchte gebunden. Dies erlaubt ein Öffnen des Containments und ein anschließendes manuelles Reinigen. Es hat sich allerdings gezeigt, dass die geometrisch komplexe Bauweise der Maschine verhindert, dass alle Bereiche durch den Sprühnebel erreicht und die dort vorzufindenden Pulverrückstände gebunden werden.

Das Dokument EP 2 883 526 A1 offenbart ein Verarbeitungssystem gemäß dem Oberbegriff des Anspruchs 1.

Der Erfindung liegt die Aufgabe zugrunde, ein gattungsgemäßes Verarbeitungssystem derart weiterzubilden, dass bei geringem Investitionsaufwand eine einfache und sichere Reinigung möglich ist.

Diese Aufgabe wird durch ein Verarbeitungssystem mit den Merkmalen des Anspruchs 1 gelöst.

Der Erfindung liegt des Weiteren die Aufgabe zugrunde, ein geeignetes Verfahren zur Dekontamination eines solchen Verarbeitungssystems anzugeben.

Diese Aufgabe wird durch ein Verfahren mit den Merkmalen des Anspruchs 6 gelöst. Nach der Erfindung ist ein Dampferzeuger als Teil des Verarbeitungssystems vorgesehen. Dieser und das zugehörige erfindungsgemäße Verfahren sind dazu ausgelegt, Wasserdampf in den Produktionsraum derart einzuleiten, dass dort zumindest abschnittsweise mit Wasserdampf übersättigte Luft entsteht. Infolge der Übersättigung kondensiert Wasser im Produktionsraum, und zwar auf Oberflächen der Verarbeitungsvorrichtung und auch in der Luft als Nebel. Durch die kondensierten Wassermengen werden eventuell auf den Oberflächen vorhandene Pulverrückstände und/oder frei in der Luft getragene Pulverkörner gebunden. Das kondensierte Wasser wird dann einschließlich der darin gebundenen Pulverrückstände entfernt, was insbesondere bei geöffnetem Gehäuse erfolgen kann.

Üblicherweise ist in der Technik die Bildung von Kondenswasser unerwünscht, was insbesondere für den technischen Bereich der Pulververarbeitung gilt. Entgegen dieser Grundregel wird nun gemäß der Erfindung eine Kondenswasserbildung bewusst herbeigeführt. Als Vorbereitung für die anschließende Reinigung macht sie sich den Umstand zunutze, dass kein gesteuerter bzw. zielgerichteter Feuchtigkeitsauftrag erfolgen muss. Vielmehr findet die Kondenswasserbildung ohne steuerndes Eingreifen in jedem noch so unzugänglichen Winkel der Maschine statt. Pulverreste, die sich insbesondere in solchen unzugänglichen Stellen ansammeln können, werden zuverlässig und lückenlos mit Wasser benetzt und dadurch gebunden. Im Anschluss an die Kondensatbildung kann also das Gehäuse bzw. das Containment der Maschine gefahrlos geöffnet werden. Es kann dann eine manuelle Reinigung erfolgen. Der Investitionsaufwand für die Dampfeinspeisung ist gering. Gleichzeitig sind abweichend von WiP-Konfigurationen keine besonderen Dichtungsmaßnahmen gegen Austreten des Waschwassers erforderlich. Eine Reinigung der Maschine ist zuverlässig, sicher und mit geringem Aufwand möglich.

In einer bevorzugten Ausführungsform ist im Produktionsraum eine ortsfeste, vom Dampferzeuger gespeiste Dampfdüse angeordnet. Hierdurch kann bei Bedarf Wasserdampf so lange eingeleitet werden, bis ein übersättigtes Luft-Dampf-Gemisch entsteht. Über diese ortsfeste Dampfdüse kann der Zustand der Übersättigung so lange aufrechterhalten werden, bis sich an den relevanten Stellen eine ausreichende Kondensatbildung eingestellt hat.

In einer weiteren vorteilhaften Ausgestaltung umfasst das erfindungsgemäße Verarbeitungssystem eine manuell zu führende Druckdüse. Hierbei kann es sich um eine Luft- oder Wasserdruckdüse oder auch um eine Zweistoffdüse handeln, mittels derer vor der eigentlichen Kondensatbildung eine Vorreinigung durchgeführt wird, indem die erreichbaren Stellen unter Druck freigeblasen werden. In bevorzugter Weiterbildung ist die genannte Druckdüse vom Dampferzeuger gespeist. Der hiermit eingetragene Wasserdampf erfährt dabei eine Doppelnutzung: Zunächst dient er aufgrund seiner Druckwirkung der Vorreinigung. Darüber hinaus trägt der eingespeiste Dampf zur Erreichung des übersättigten Zustandes und damit zur Kondensatbildung bei. Die dampfbeaufschlagte Druckdüse kann damit als Ergänzung zur ortsfesten Dampfdüse, aber auch als deren Ersatz eingesetzt werden.

Zusätzlich kann es zweckmäßig sein, einen Ultraschallbefeuchter vorzusehen, mittels dessen feine Wassertröpfchen in den geschlossenen Produktionsraum eingetragen werden, und wodurch die durch kondensiertes Wasser hervorgerufene Bindung von Pulverrückständen unterstützt wird.

Ein Ausführungsbeispiel der Erfindung ist nachfolgend anhand der Zeichnung näher beschrieben.

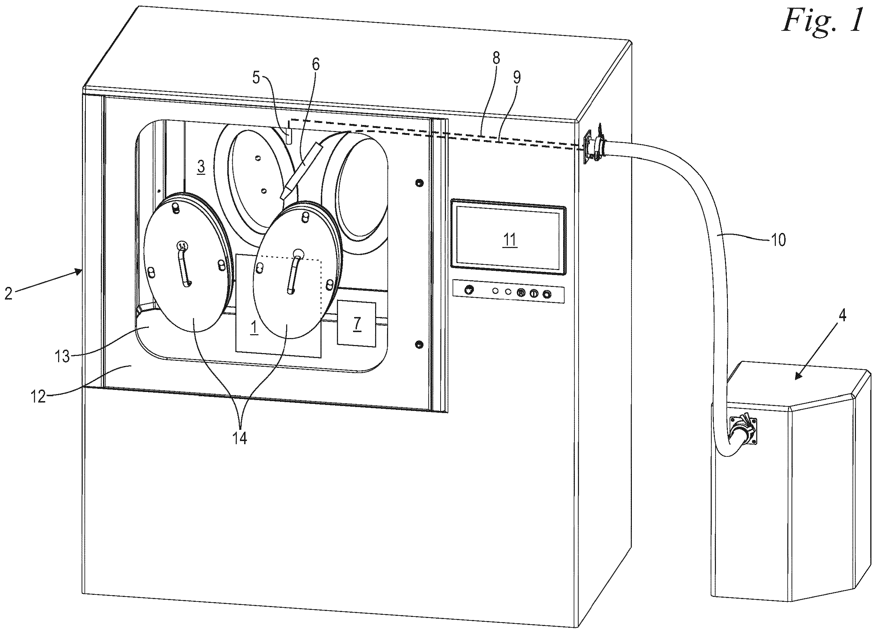

Fig. 1 zeigt in einer perspektivischen Ansicht ein erfindungsgemäß ausgeführtes Verarbeitungssystem für pharmazeutische Pulver. Das Verarbeitungssystem umfasst eine nur schematisch als Block angedeutete Verarbeitungsvorrichtung 1 für das Pulver, welche hier eine Kapselfülleinrichtung ist, aber auch eine Tablettenpresse oder dgl. sein kann. Des Weiteren umfasst das Verarbeitungssystem ein geschlossenes Gehäuse 2, welches einen inneren Produktionsraum 3 umschließt und diesen während des Betriebes hermetisch gegenüber der äußeren Umgebung abdichtet. Die Verarbeitungsvorrichtung 1 ist in diesem inneren Produktionsraum 3 platziert, so dass keine Teilmengen des zu verarbeitenden Pulvers nach außen gelangen können.

Eine Bedienung der Verarbeitungsvorrichtung 1 von der Außenseite des Gehäuses 2 aus ist mittels eines Nutzerinterfaces 11 möglich. Eine visuelle Überwachung des Betriebes kann durch ein Fenster 13 vorgenommen werden, welches zudem mit Handschuheingriffen 14 ausgestattet ist. Bei Bedarf kann ein manueller Zugriff über die Handschuheingriffe 14 erfolgen, ohne dass das Gehäuse 2 geöffnet werden muss. Im dekontaminierten Zustand kann das Gehäuse 2 jedoch geöffnet werden, wozu eine Tür 12 vorgesehen ist.

Während des Betriebes der Verarbeitungsvorrichtung 1 können sich im inneren Produktionsraum 3 auf den Innenflächen des Gehäuses 2 sowie auf allen Oberflächen der Verarbeitungsvorrichtung 1 Pulverrückstände absetzen. Außerdem schweben in der Luft des Produktionsraumes 3 luftgetragene Pulverpartikel. All diese Pulverrückstände bzw. Pulverpartikel müssen bei Bedarf durch Reinigen entfernt werden. Hierzu umfasst das erfindungsgemäße Verarbeitungssystem neben dem Gehäuse 2 und der im inneren Produktionsraum 3 angeordneten Verarbeitungsvorrichtung 1 zusätzlich noch einen Dampferzeuger 4. Der Dampferzeuger 4 ist als eigenständige Einheit separat vom Gehäuse 2 dargestellt, kann aber auch in das Gehäuse 2 bzw. in die Verarbeitungsvorrichtung 1 integriert sein. Eine Ansteuerung des Dampferzeugers 4 erfolgt über das Nutzerinterface 11. Der Dampferzeuger 4 ist über einen Medienschlauch 10 mit dem Gehäuse 2 verbunden. Im Betrieb, d. h. für den Zweck einer Dekontamination nach Produktionsende oder in einer Produktionspause, wird mittels des Dampferzeugers 4 heißer Wasserdampf, also Wasser in seiner gasförmigen Phase erzeugt und durch den Medienschlauch 10 in den Produktionsraum 3 eingeleitet. Der Dampferzeuger 4 ist hinsichtlich seiner Kapazität und Ansteuerung dazu ausgelegt, dass im Produktionsraum 3 mit Wasserdampf übersättigte Luft entsteht. Im entsprechenden Verfahrensschritt wird also Wasserdampf in einer solchen Menge in den Produktionsraum 3 eingeleitet, dass die ohnehin darin vorhandene Luft mit Wasserdampf übersättigt wird und folglich zu einem Aufkondensieren von Wasser führt. Das Kondensieren des Wassers erfolgt an allen freien Oberflächen im Produktionsraum 3, also als Nebeltröpfchen an frei in der Luftfüllung des Produktionsraumes schwebenden Pulverpartikeln, an den Innenflächen des Gehäuses 2 sowie an allen freien Oberflächen der Verarbeitungsvorrichtung 1. Dabei wird so lange Wasserdampf nachgeführt, bis alle Schwebeteilchen von Nebeltröpfchen erfasst und bis alle Oberflächen mit Kondenswasser in flüssiger Phase soweit benetzt sind, dass daran anhaftende Pulverrückstände durch das Wasser gebunden sind. Die Nebeltröpfchen mit den darin gebundenen Pulverpartikeln sedimentieren nach einer Weile, setzen sich also an den Innenflächen des Gehäuses 2 sowie an den freien Oberflächen der Verarbeitungsvorrichtung 1 ab.

Nach erfolgter Kondensation und Sedimentierung, also nach Bindung der Pulverrückstände kann die Tür 12 geöffnet werden und eine Reinigung des Produktionsraumes 3 einschließlich der Verarbeitungsvorrichtung 1 erfolgen. Hierzu kann das Kondensat mit den darin gebundenen Pulverrückständen abgesaugt und/oder abgewischt werden.

Die Einleitung des Wasserdampfes in den Produktionsraum 3 erfolgt im gezeigten Ausführungsbeispiel mittels einer ortsfesten, vom Dampferzeuger 4 durch eine Dampfleitung 8 gespeisten Dampfdüse. Der hierdurch eingespeiste Wasserdampf durchmischt sich mit der Luft, bis die gewünschte Übersättigung zumindest abschnittsweise, das heißt in zumindest allen relevanten Volumenbereichen erreicht ist. Zur Unterstützung des dann einsetzenden Kondensationsprozesses und der damit einhergehenden Feuchtigkeitsbenetzung der Oberflächen kann optional ein Ultraschallbefeuchter 7 eingesetzt werden, welcher hier nur in schematischer Blockdarstellung angedeutet und im Produktionsraum 3 positioniert ist. Der Ultraschallbefeuchter 7 kann aber auch an anderer geeigneter Stelle, beispielsweise im Gehäuse des Dampferzeugers 4 angeordnet sein. Jedenfalls können mittels des Ultraschallbefeuchters 7 bei Bedarf feine Wassertröpfchen erzeugt und in die Luftfüllung des Produktionsraumes 3 abgegeben werden. Auch diese Wassertröpfchen binden Schwebeteilchen, und auch sie setzen sich auf den freien Oberflächen ab und tragen zur Bindung von Pulverrückständen bei.

Vor dem beschriebenen Prozess der Pulverbindung mittels kondensiertem Wasser kann optional eine Vorreinigung der Oberflächen bei geschlossenem Gehäuse 2 erfolgen. Hierzu ist optional eine Druckdüse 6 vorgesehen, welche durch einen flexiblen Schlauch gespeist ist und mittels der Handschuheingriffe 14 manuell geführt werden kann. Der Maschinenbediener kann hiermit die verschmutzten Oberflächen freiblasen, woraufhin dann die aufgewirbelten Pulvermengen abgesaugt werden. Die Druckdüse 6 kann beispielsweise eine Druckluftdüse oder eine Mehrstoffdüse zur Einspeisung eines Luft-Wasser-Gemisches sein. Im gezeigten Ausführungsbeispiel handelt es sich um eine Heißdampfdüse, welche durch eine Dampfleitung 9 vom Dampferzeuger 4 gespeist ist.

Der mit Druck auf die entsprechenden Oberflächen auftreffende Heißdampf führt zunächst in vorstehend beschriebener Weise zu einer Vorreinigung. Darüber hinaus trägt er zur Erhöhung der relativen Luftfeuchte im Produktionsraum 3 bei. Dementsprechend muss anschließend nur noch eine geringere Menge von Wasserdampf durch die ortsfeste Dampfdüse 5 eingespeist werden, bis der Zustand der Übersättigung und Kondensation eintritt. Alternativ kann es auch ausreichen, auf die ortsfeste Dampfdüse 5 ganz zu verzichten und die Wasserdampfeinspeisung sowohl für die Vorreinigung als auch für die Erzeugung des übersättigten Kondensationszustandes allein mittels der Druckdüse 6 vorzunehmen.

## Patentansprüche

1. Verarbeitungssystem für insbesondere pharmazeutische Pulver, umfassend eine Verarbeitungsvorrichtung (1) für das Pulver sowie ein geschlossenes Gehäuse (2) mit einem inneren Produktionsraum (3), wobei die Verarbeitungsvorrichtung (1) in dem vom Gehäuse (2) umschlossenen Produktionsraum (3) angeordnet ist, **dadurch gekennzeichnet, dass** das Verarbeitungssystem des Weiteren einen Dampferzeuger (4) umfasst, welcher dazu ausgelegt ist, Wasserdampf in den Produktionsraum (3) derart einzuleiten, dass dort zumindest abschnittsweise mit Wasserdampf übersättigte Luft entsteht.

2. Verarbeitungssystem nach Anspruch 1,
**dadurch gekennzeichnet, dass** im Produktionsraum (3) eine ortsfeste, vom Dampferzeuger (4) gespeiste Dampfdüse (5) angeordnet ist.

3. Verarbeitungssystem nach Anspruch 1 oder 2,
**dadurch gekennzeichnet, dass** das Verarbeitungssystem eine manuell zu führende Druckdüse (6) umfasst.

4. Verarbeitungssystem nach Anspruch 3,
**dadurch gekennzeichnet, dass** die Druckdüse (6) vom Dampferzeuger (4) gespeist ist.

5. Verarbeitungssystem nach einem der Ansprüche 1 bis 4,
**dadurch gekennzeichnet, dass** das Verarbeitungssystem zusätzlich einen Ultraschallbefeuchter (7) zur Einleitung von Feuchtigkeit in den Produktionsraum (3) umfasst.

6. Verfahren zur Dekontamination eines Verarbeitungssystems nach einem der Ansprüche 1 bis 5, umfassend folgende Verfahrensschritte:
- Bei geschlossenem Gehäuse (2) wird in den Produktionsraum (3) Wasserdampf in einer solchen Menge eingeleitet, dass dort zumindest abschnittsweise mit Wasserdampf übersättigte Luft entsteht.
- Eventuell vorhandene Pulverrückstände werden mittels Wasser dadurch gebunden, dass infolge der Übersättigung Wasser im Produktionsraum (3) kondensiert.
- Das kondensierte Wasser einschließlich darin gebundener Pulverrückstände wird bei insbesondere geöffnetem Gehäuse (2) entfernt.

7. Verfahren nach Anspruch 6,
**dadurch gekennzeichnet, dass** bei geschlossenem Gehäuse (2) eine Vorreinigung mittels einer insbesondere vom Dampferzeuger (4) gespeisten Druckdüse (6) vorgenommen wird.

8. Verfahren nach Anspruch 6 oder 7,
**dadurch gekennzeichnet, dass** die durch kondensiertes Wasser hervorgerufene Bindung von Pulverrückständen mittels eines Ultraschallbefeuchters (7) unterstützt wird.

## Claims

1. Processing system for in particular pharmaceutical powders, comprising a processing device (1) for the powder, and a closed housing (2) with an inner production space (3), the processing device (1) being arranged in the production space (3) enclosed by the housing (2),
**characterized in that** the processing system further comprises a steam generator (4), which is designed to introduce water vapour into the production space (3) in such a way that air supersaturated with water vapour develops there at least in some parts.

2. Processing system according to claim 1,
**characterized in that** a stationary steam nozzle (5), supplied from the steam generator (4), is arranged in the production space (3).

3. Processing system according to claim 1 or 2,
**characterized in that** the processing system comprises a pressure nozzle (6), which is to be guided manually.

4. Processing system according to claim 3,
**characterized in that** the pressure nozzle (6) is supplied from the steam generator (4).

5. Processing system according to one of claims 1 to 4,
**characterized in that** the processing system additionally comprises an ultrasonic humidifier (7) for introducing moisture into the production space (3).

6. Method for decontamination of a processing system according to one of claim 1 to 5, comprising the following method steps:
- With the housing (2) closed, water vapour is introduced into the production space (3) in such a quantity that air supersaturated with water vapour develops there at least in some parts.
- Any powder residues present are bound by means of water since, on account of the supersaturation, water condenses in the production space (3).
- The condensed water, including powder residues bound therein, is removed, in particular with the housing (2) opened.

7. Method according to claim 6,
**characterized in that** preliminary cleaning by means of a pressure nozzle (6), in particular supplied from the pressure generator (4), is performed with the housing (2) closed.

8. Method according to claim 6 or 7,
**characterized in that** the binding of powder residues, which is brought about by condensed water, is supported by means of an ultrasonic humidifier (7).

## Revendications

1. Système de traitement pour poudre en particulier pharmaceutique, comprenant un dispositif de traitement (1) pour la poudre ainsi qu'un boîtier fermé (2) doté d'un espace de production intérieur (3), le dispositif de traitement (1) étant disposé dans l'espace de production (3) entouré par le boîtier (2),
**caractérisé en ce que** le système de traitement comprend en outre un générateur de vapeur (4), lequel est conçu pour introduire de la vapeur d'eau dans l'espace de production (3), de telle sorte que de l'air sursaturé en vapeur d'eau s'y forme au moins dans certaines parties.

2. Système de traitement selon la revendication 1,
**caractérisé en ce qu'**une buse à vapeur (5) fixée à demeure et alimentée par le générateur de vapeur (4) est disposée dans l'espace de production (3).

3. Système de traitement selon la revendication 1 ou 2,
**caractérisé en ce que** le système de traitement comprend une buse à pression (6) devant être guidée manuellement.

4. Système de traitement selon la revendication 3,
**caractérisé en ce que** la buse à pression (6) est alimentée par le générateur de vapeur (4).

5. Système de traitement selon l'une des revendications 1 à 4,
**caractérisé en ce que** le système de traitement comprend en outre un humidificateur à ultrasons (7) servant à l'introduction d'humidité dans l'espace de production (3).

6. Procédé de décontamination d'un système de traitement selon l'une des revendications 1 à 5, comprenant les étapes de procédé suivantes :
- lorsque le boîtier (2) est fermé, de la vapeur d'eau est introduite dans l'espace de production (3) dans une quantité telle que de l'air sursaturé en vapeur d'eau s'y forme au moins dans certaines parties,
- des résidus de poudre éventuellement présents sont liés au moyen de l'eau de telle sorte que, du fait de la sursaturation, l'eau se condense dans l'espace de production (3),
- l'eau condensée, y compris les résidus de poudre liés dans celle-ci, est retirée lorsque le boîtier (2) est en particulier ouvert.

7. Procédé selon la revendication 6,
**caractérisé en ce que**, lorsque le boîtier (2) est fermé, un nettoyage préliminaire est effectué au moyen d'une buse à pression (6) alimentée en particulier par le générateur de vapeur (4).

8. Procédé selon la revendication 6 ou 7,
**caractérisé en ce que** la liaison, provoquée par l'eau condensée, de résidus de poudre est favorisée au moyen d'un humidificateur à ultrasons (7).
